(19) Europäisches Patentamt

European Patent Office

Office européen des brevets

(11) **EP 0 629 403 B1**

(12) **EUROPÄISCHE PATENTSCHRIFT**

(45) Veröffentlichungstag und Bekanntmachung des Hinweises auf die Patenterteilung:
**09.09.1998 Patentblatt 1998/37**

(51) Int. Cl.$^6$: **A61K 31/505**
// (A61K31/505, 31:135)

(21) Anmeldenummer: **94108767.8**

(22) Anmeldetag: **08.06.1994**

(54) **Antibiotische Kombination**

Antibiotic combination

Mélange d'antibiotiques

(84) Benannte Vertragsstaaten:
**AT BE CH DE DK ES FR GB GR IE IT LI LU MC NL PT SE**

(30) Priorität: **17.06.1993 CH 1801/93**

(43) Veröffentlichungstag der Anmeldung:
**21.12.1994 Patentblatt 1994/51**

(73) Patentinhaber:
**F. HOFFMANN-LA ROCHE AG**
**4002 Basel (CH)**

(72) Erfinder:
• **Kompis, Ivan**
**CH-4104 Oberwil (CH)**

• **Then, Rudolf**
**D-79576 Weil am Rhein (DE)**

(74) Vertreter:
**Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**WO-A-92/08461**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein antibiotisch wirksames Kombinationspräparat bestehend aus 2,4-Diamino-5-[3,5-diäthoxy-4-(pyrrol-1-yl)-benzyl]pyrimidin (Epiroprim) und 4,4'-Diamino-diphenylsulfon (Dapson). Die Erfindung betrifft ferner die Verwendung von Epiroprim und Dapson als Wirkstoffe bei der Herstellung von pharmazeutischen Präparaten zur Behandlung von Infektionen mit Mycobacterien, Actinomyceten insbesondere Nocardia sp., und Toxoplasmose-Erregern.

Die Anwendung von Diaminobenzylpyrmidinen, einschliesslich Epiroprim, gegebenenfalls in Kombination mit Dapson oder antibakteriell wirksamen Sulfonamiden, gegen Infektionen mit dem Pilz Pneumocystis carinii ist aus der Patentpublikation WO 92 08461 bekannt. Ueberraschend wurde gefunden, dass sich die Kombination von Epiroprim mit Dapson zur Behandlung von Infektionen mit Mycobacterien, insbesondere M. marinum, M. smegmatis, M. avium und M. leprae; und von Toxoplasmose-Erregern, insbesondere Toxoplasma gondii; sowie Actinomyceten, insbesondere Nocardia sp. eignet.

Die Wirkstoffe können einzeln oder als Kombinationspräparat, und gleichzeitig oder zeitlich abgestuft verabreicht werden, wobei die gleichzeitige Verabreichung bevorzugt ist.

Die Wirkstoffe können enteral, parenteral oder topisch verabreicht werden. Für die enterale Applikation eignen sich z.B. Tabletten, Kapseln, Dragées, Sirupe, Suspensionen, Lösungen und Suppositorien. Für die parenterale Applikation sind Infusions- oder Injektionslösungen geeignet.

Die Dosierungen, in denen die Wirkstoffe verabreicht werden, können je nach Anwendungsart und Anwendungsweg sowie nach den Bedürfnissen der Patienten variieren.

Zur Bekämpfung der vorstehend genannten Infektionen bzw. Erreger können die Wirkstoffe im Verhaltnis von etwa 1:100 bis etwa 100:1 Gew.-Teilen Epiroprim:Dapson eingesetzt werden.

Bei der Behandlung von Infektionen mit Mycobacterien ist ein Verhältnis Epiroprim:Dapson von etwa 1:1 bis etwa 1:5 Gew.-Teilen bevorzugt. Bei der Behandlung von Infektionen mit Toxoplasma-Erregern ist ein Verhältnis von etwa 1:2 bis 2:1, bei Nocardia-Infektionen ein Verhältnis von etwa 4:1 bis etwa 1:1 Gew.-Teilen Epiroprim:Dapson bevorzugt.

Die Dosierung der Wirkstoffe beträgt bei oraler Verabreichung zweckmässig etwa 0,5 bis 50 mg/kg Körpergewicht pro Tag, vorzugsweise 0,5-3 mg/kg Körpergewicht pro Tag für Epiroprim; und etwa 0,5-2 mg/kg Körpergewicht pro Tag für Dapson.

In Dosierungsformen, z.B. Tabletten, können die Wirkstoffe z.B. in Mengen von 20-200 mg Epiroprim und 20-100 mg Dapson pro Tablette enthalten sein.

Die Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmes, Lösungen, Lotionen, Sprays, Suspensionen und dergleichen verwendet. Bevorzugt sind Salben und Crèmes sowie Lösungen. Diese zur topischen Anwendung bestimmten Präparate können dadurch hergestellt werden, dass mm die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben oder Crèmen geeignet.

Den Präparaten kann gegebenenfalls ein Antioxydationsmittel, z.B.Tocopherol, N-Methyl-$\gamma$-tocopheramin sowie t-Butyl-hydroxyanisol oder t-Butyl-hydroxytoluol beigemischt werden.

Die Wirksamkeit der erfindungsgemässen Wirkstoffkombination ist aus den in den nachstehenden Tabellen angeführten Daten ersichtlich.

In den Tabellen 1 und 2 ist die in-vivo Wirksamkeit der Wirkstoffe einzeln und in Kombination als minimale Hemmkonzentration (MHK) für verschiedene Mycobacterien- und Actinomycetenstämme sowie der FIC (fractionary inhibitory concentration)-Index angegeben, der ein Mass für den synergistischen Effekt darstellt (vgl. J. Biol. Chem. 1954, 208, 477). Je kleiner der FIC-Index ist, desto stärker ist der synergistische Effekt.

Tabelle 3 zeigt die mit bestimmten Konzentrationen der einzelnen Wirkstoffe und deren kombinierten Anwendung erreichten prozentualen Hemmung von M. lufu-Kulturen, die ein anerkanntes Modell für die Prüfung von Heilmitteln gegen Lepra darstellen.

Die Tabellen 4 und 5 zeigen die in-vivo Wirksamkeit bei Nocardia- und Toxoplasma-Infektionen. Die Versuchstiere wurden i.p. mit 10 000 Koloniebildenden Einheiten des Erregers RH Toxoplasma gondii infiziert. 24 Stunden nach der Infektion wurden den Tieren während 14 Tagen zweimal täglich die angegebenen Wirkstoffdosen durch Schlundsonde verabreicht. Als "geheilt" werden die Tiere ohne nachweisbare Cysten im Hirn bezeichnet.

## Tabelle 1

### Checkerboard-Titration gegen verschiedene Mycobacterien

| Erreger | MHK allein | | MHK in Kombination | | FIC Indizes |
|---|---|---|---|---|---|
| | Dapson [μg/ml] | Epiroprim | Dapson + [μg/ml] | Epiroprim | |
| M. avium SN 304 | >30 | >30 | 6 | 15 | <0.7 |
| | | | 3 | 18 | <0.7 |
| M. intracellulare SN 403 | >30 | 8 | 3 | 1 | >0.23 |
| M. kansasii | >20 | >20 | 8 | 10 | <0.9 |
| | | | 6 | 11 | <0.9 |
| M. lufu L 209 | 0.012 | > 6 | 0.002 | 1.2 | <0.36 |
| M. marinum SN 1254 | 3 | 16 | 0.5 | 2 | ≤0.3 |
| M. smegmatis ATCC 607 | 0.4 | 2.5 | 0.08 | 0.5 | ≤0.4 |

EP 0 629 403 B1

## Tabelle 2

### In-vitro Wirksamkeit gegen Actinomyceten

| Erreger | Inkubationszeit (Tage) | MHK allein Dapson [µg/ml] | Epiroprim | MHK in Kombination Dapson + Epiroprim [µg/ml] | | FIC-Indizes |
|---|---|---|---|---|---|---|
| Nocardia asteroides N1 | 3 | >100 | 100 | 3.125 | 3 | 0.0625 |
| | 7 | >100 | >100 | 6 | 6 | 0.125 |
| Nocardia asteroides N10 | 3 | 25 | 25 | <3 | <3 | <0.25 |
| | 7 | 50 | 50 | 3 | 3 | 0.125 |
| Nocardia brasiliensis N2 | 7 | 12.5 | >100 | 6.25 | 6.25 | 0.53 |
| Streptomyces sp. N31 | 3 | >100 | 25 | 3 | 6 | <0.281 |
| | 7 | >100 | 100 | 3 | 12 | <0.186 |
| Actinomadura madurae N3 | 7 | >100 | >100 | 50 | 50 | <0.5 |

Tabelle 3

| Kombination von Epiroprim mit Dapson in vitro gegen Mycobacterium lufu | | |
|---|---|---|
| | µg/ml | % Hemmung |
| Epiroprim | 5 | 14 |
| | 25 | 34 |
| | 60 | 73 |
| Dapson | 0.2 | 22 |
| | 0.4 | 22 |
| Epiroprim + Dapson | 0.2 + 5 | 84 |
| | 0.4 + 25 | 96 |

Tabelle 4

| Wirksam in vivo gegen Nocardiosis bei Mäusen | | | |
|---|---|---|---|
| Dosierung [mg/kg] | | % Ueberlebende | Mittlere Ueberlebensdauer (Tage) |
| Dapson | Epiroprim | | |
| 1 | - | 0 | 2,6 |
| - | 30 | 0 | 1,7 |
| - | 100 | 0 | 2 |
| 1 | 30 | 20 | 6,6 |
| 1 | 100 | 40 | 13,2 |
| 3 | 30 | 60 | 12,2 |
| 3 | 100 | 100 | >20 |

Tabelle 5

| In-vivo-Aktivität gegen Toxoplasmose | | | |
|---|---|---|---|
| | Dosis (mg) | Anzahl Ueberlebende | geheilte Tiere in % der Ueberlebenden |
| Dapson | 50 | 1/10 | 0 |
| Dapson | 100 | 10/10 | 0 |
| Epiroprim | 100 | 0/10 | - |
| Kombination Epiroprim/Dapson | 25/25 | 0/10 | |
| | 50/50 | 10/10 | 30 |
| | 100/50 | 10/10 | 20 |
| | 100/100 | 10/10 | 50 |
| Kontrolle | 0/30 | 0/30 | |

Die erfindungsgemässe Wirkstoffkombination kann z.B. zur Behandlung von Nocardiosen, Actinomycetosen und Mycetomen, verursacht durch Actinomyceten, wie Actinomyces israeli, A. naeslundii u.a., Norcardia brasiliensis, N. asteroides, u.a. sowie durch Streptomyceten wie Streptomyces madurae, S. someliensis, S. pelletieri, Madurella mycetomi; generalisierten Infektionen mit Mycobacterien, insbesondere solchen mit M. avium und M. intracellulare bei Patienten mit beeinträchtigten Funktionen des Immunsystems; sowie zur Behandlung von Lepra und cerebraler Toxoplasmose verwendet werden.

Ein Beispiel einer typischen Dosierungsform ist die folgende:

| Tablette | |
|---|---|
| Dapson | 100 mg |
| Epiroprim | 200 |
| PRIMOJEL (Stärke-Derivat) | 6 |
| Povidone K 30 (Polyvinylpyrrolidon) | 8 |
| Magnesiumstearat | 6 |
| Totalgewicht | 320 mg |

## Patentansprüche

1. Pharmazeutische Präparate, enthaltend Epiroprim und Dapson, sowie übliche pharmazeutische Trägerstoffe.

2. Präparate gemäss Anspruch 1, enthaltend Epiroprim und Dapson im Verhältnis von 1:100 bis 100:1 Gew.-Teilen.

3. Präparat gemäss Anspruch 1, enthaltend Epiroprim und Dapson im Verhältnis von 1:1 bis 1:5 Gew.-Teilen.

4. Verwendung von Epiroprim und Dapson zur Herstellung von pharmazeutischen Präparaten zur Behandlung von Infektionen mit Mycobacterien, Actinomyceten, insbesondere Nocardia sp., und Toxoplasmose-Erregern.

## Claims

1. Pharmaceutical preparations containing epiroprim and dapsone as well as usual pharmaceutical carriers.

2. Preparations according to claim 1 containing epiroprim and dapsone in the ratio of 1:100 to 100:1 parts by weight.

3. Preparations according to claim 1 containing epiroprim and dapsone in the ratio of 1:1 to 1:5 parts by weight.

4. The use of epiroprim and dapsone for the production of pharmaceutical preparations for the treatment of infections with mycobacteria, Actinomycetes, especially Nocardia sp., and toxoplasmosis pathogens.

## Revendications

1. Compositions pharmaceutiques, contenant de l'épiroprim et de la dapsone, ainsi que des véhicules pharmaceutiques usuels.

2. Compositions selon la revendication 1, contenant de l'épiroprim et de la dapsone en un rapport allant de 1 : 100 à 100:1 parties en poids

3. Compositions selon la revendication 1, contenant de l' épiroprim et de la dapsone en un rapport allant de 1 : 1 à 1:5 parties en poids.

4. Utilisation d'épiroprim et de dapsone pour la préparation de compositions pharmaceutiques destinées au traitement d'infections à mycobactéries, actinonmycètes, en particulier Nocardia sp., et agents pathogènes de la toxoplasmose.